**Europäisches Patentamt**

**European Patent Office**

**Office européen des brevets**

(19)

(11) Veröffentlichungsnummer: **0 319 754 B1**

(12)

# EUROPÄISCHE PATENTSCHRIFT

(45) Veröffentlichungstag der Patentschrift:
24.07.91 Patentblatt 91/30

(51) Int. Cl.⁵: **B01J 27/192**, C07C 51/25, C07C 57/04

(21) Anmeldenummer: 88119197.7

(22) Anmeldetag: 18.11.88

(54) **Verfahren zur Herstellung einer für die Gasphasenoxidation von Propylen zu Acrolein und Acrylsäure katalytisch aktiven Masse enthaltend Mo, Bi, Fe, Co u./o. Ni.**

(30) Priorität: 27.11.87 DE 3740271

(43) Veröffentlichungstag der Anmeldung:
14.06.89 Patentblatt 89/24

(45) Bekanntmachung des Hinweises auf die
Patenterteilung:
24.07.91 Patentblatt 91/30

(84) Benannte Vertragsstaaten:
DE ES FR GB IT

(56) Entgegenhaltungen:
GB-A- 1 282 949
US-A- 4 168 246
US-A- 4 190 608
US-A- 4 267 386
US-A- 4 272 637
US-A- 4 306 090

(73) Patentinhaber: BASF Aktiengesellschaft
Carl-Bosch-Strasse 38
W-6700 Ludwigshafen (DE)

(72) Erfinder: Martan, Hans, Dr.
Mozartstrasse 62
W-6710 Frankenthal (DE)
Erfinder: Schwarzmann, Matthias, Dr.
Carl-Bosch-Strasse 54
W-6703 Limburgerhof (DE)
Erfinder: Boehning, Karl-Heinz, Dr.
Mecklenburger Strasse 14
W-6100 Darmstadt (DE)
Erfinder: Dreyer, Heinz, Dr.
Parkstrasse 18
W-6700 Ludwigshafen (DE)

## Beschreibung

Diese Erfindung betrifft ein spezielles Verfahren zur Herstellung einer für die Gasphasenoxidation von Propylen zu Acrolein und Acrylsäure katalytisch aktiven Masse auf Basis von Oxiden oder Mischoxiden aus Molybdän, Wismut, Eisen und anderen hierfür üblichen metallischen Komponenten.

Katalytisch aktive Massen der allgemeinen Formel

$$Mo_{12}Bi_{0,1-10}Fe_{0,8-12}[Co,Ni]_{0,1-10}P_{0-2}A_{0-10}B_{0,001-1}O_x,$$

in der A für die Elemente Arsen, Antimon, Zinn, Thallium, Wolfram, die Erdalkalimetalle, Zink und/oder Chrom, B für mindestens eines der Metalle Natrium, Kalium, Rubidium, Cäsium und/oder Indium und X für die durch die Atomwertigkeiten der Einzelelemente bestimmte Zahl stehen, sind beispielsweise aus der DE-OS 33 38 380, der GB-PS 1 437 235 (O.Z. 0050/39443) und der DE-PS 2 229 358 bekannt. Sie werden im allgemeinen durch Vermischen von wasserlöslichen Salzen der Bestandteile im wäßrigem Medium, Abdampfen des Wassers und Kalzinieren bei Temperaturen von 400 bis 650°C hergestellt. Bei dem Verfahren der DE-OS 33 38 380 kann dabei der Gehalt der katalytisch aktiven Masse an Wismut und Woffram dem Gemisch der wasserlöslichen Salze der anderen Komponenten zugegeben und hierdurch eine verhältnismäßig niedrige Reaktionstemperatur bei der Gasphasenoxidation von Propylen bei gleichzeitiger Erhöhung der Gesamtausbeute an Acrolein und Acrylsäure erzielt werden. Bei dem Verfahren gemäß US-A-4168246 werden wäßrige Suspensionen von Co—, Ni— u/o Fe— molyldat mit Wismut oxid (oder einem Salt), ggf. einem Phosphatrest, einem Ion aus der Gruppe Na, K und Ca und mit Kieselsol versetzt.

Das Abdampfen des Wassers aus dem wäßrigen Gemisch der Salze der Katalysatorkomponenten kann beispielsweise durch Abdampfen unter Rühren der Massen erfolgen, doch fallen dabei die einzelnen Komponenten in Abstufung ihres Löslichkeitsproduktes aus der Lösung aus und es kann auf diese Weise keine homogene Mischung des getrockneten Materials erreicht werden, selbst wenn dieses noch weitgehend gemahlen wird. Auch beim Sprühtrocknen der wäßrigen Gemische der Salze der Komponenten, wie es z.B. in der DE-PS 2 229 358 beschrieben ist, treten trotz Beachtens vieler Randbedingungen Probleme durch Verkleben der Düsen, Anbacken des Produkts an den Wänden und "Brockenbildung" auf und die Sprühprodukte müssen selbst bei optimalem Verlauf der Trocknung meist noch zu größeren Feststoffteilen agglomeriert werden, um bei der weiteren Verarbeitung ein Stauben zu vermeiden und die Handhabung zu verbessern.

Aufgabe der vorliegenden Erfindung ist nun, ein Verfahren zur Herstellung einer derartigen katalytisch aktiven Masse, in der die Komponenten besonders homogen verteilt sind und eine einfache Handhabung gewährleistet ist, zur Verfügung zu stellen.

Es wurde nun gefunden, daß man katalytisch aktive Massen für die Gasphasenoxidation von Propylen zu Acrolein und Acrylsäure der allgemeinen Formel

$$Mo_{12}Bi_{0,1-10}Fe_{0,8-12}[Co,Ni]_{0,1-10}P_{0-2}A_{0-10}B_{0,001-1}O_x,$$

in der A für die Elemente Arsen, Antimon, Zinn, Thallium, Wolfram, die Erdalkalimetalle, Zink und/oder Chrom, B für mindestens eines der Metalle Natrium, Kalium, Rubidium, Cäsium und/oder Indium und X für die durch die Atomwertigkeiten der Einzelelemente bestimmte Zahl stehen, durch Vermischen von wasserlöslichen Salzen der Bestandteile in wäßrigen Medium, Abdampfen des Wassers und Kalzinieren bei Temperaturen von 400 bis 650°C mit Vorteil herstellen kann, indem man ein getrocknetes pulverförmiges Eisenmolybdat-Gel $(Fe(MoO_4)_3)$ zu einer wäßrigen Lösung oder Suspension von hierfür üblichen wasserlöslichen Salzen der anderen Bestandteile, einschließlich eines eventuellem Überschusses an Eisen- oder Molybdänsalz über die durch das Eisenmolybdat eingetragene Menge gibt und das Gemisch unter Rühren und eventuellem Erwärmen zum gelartigen Erstarren bringt, das Gel zerkleinert und bei erhöhter Temperatur trocknet und dann kalziniert.

Die vorherige Zubereitung von getrocknetem, pulverförmigem Eisenmolybdat-Gel kann in an sich bekannter Weise z.B. nach den Angaben der GB-PSen 1 282 949 und 1 282 950 erfolgen. Man geht dabei praktisch so vor, daß man in eine gekühlte Lösung von Ammoniumheptamolybdat langsam unter Rühren eine Eisennitratlösung gibt, weiterrührt, bis sich der zuerst gebildete Niederschlag auflöst und die wieder klare Lösung zu einem grünlichen Gel erstarrt. Dieses Gel wird zu einem rotbraunen Pulver getrocknet.

Das getrocknete pulverförmige Eisenmolybdat-Gel hat vorzugsweise eine mittlere Teilchengröße von 10 µm bis 2 mm und wird bei dem erfindungsgemäßen Verfahren im allgemeinen bei Raumtemperatur in die wäßrige Lösung bzw. Suspension der anderen Komponenten unter Rühren eingetragen. Dabei hat sich eine Rührgeschwindigkeit von 20 bis 200 upm mit einem üblichen Blattrührer besonders bewährt.

Zur Zubereitung der wäßrigen Lösung bzw. Suspension der anderen Katalysatorkomponenten können die hierfür üblichen wasserlöslichen Salze eingesetzt werden, z.B. Nitrate, Carbonate, Formiate, Acetate und Ammoniumsalze.

Soweit für die katalytisch aktive Masse mehr als 8 Mol Eisen je 12 Mol Molybdän eingesetzt werden, wird der Überschuß in Form des hierfür üblicherweise eingesetzten wasserlöslichen Eisensalzes, z.B. Eisen-III-nitrat oder Carbonat bei der Zubereitung der wäßrigen Lösung der "anderen" Komponenten zugegeben. Ist für die katalytisch aktive Masse ein Eisengehalt unter etwa 7,5 Mol Eisen auf 12 g Mol Molybdän vorgesehen, so wird der überschüssige Anteil an Molybdän bei der Bereitung der wäßrigen Lösung der "anderen" Komponenten in Form der hierfür üblichen Molybdän-Verbindungen, z.B. Ammoniumheptamolybdat oder Molybdäntrioxid, sowie ferner Ammoniumdimolybdat zugegeben. Mit anderen Worten : soll der Katalysator auf 12 Mol Molybdän z.B. 6 Mol Eisen enthalten, so wird die Gesamtmenge des Eisens in Form des Eisenmolybdat-Gels zugegeben, und 3 Mol Molybdän in Form der wasserlöslichen Molybdänverbindung. Nach dem Eintragen des pulverförmigen getrockneten Eisenmolybdats in die wäßrige Lösung bzw. Suspension der anderen Komponenten unter Rühren bildet sich, ggf. nach leichtem Erwärmen beispielsweise auf 25 bis 90, insbesondere von 30 bis 50°C, ein körniges Gel, in dem die Katalysatorkomponenten optimal homogen verteilt sind. Das körnige Gel kann beispielsweise in einem Drehrohrofen oder in einer Wirbelschicht im allgemeinen bei Temperaturen von 90 bis 350°C, vorzugsweise von 110 bis 120°C ggf. unter Überleiten von Luft getrocknet werden. Man erhält dann eine feinkörnige Masse, die leicht auf eine durchschnittliche Teilchengröße von 10 bis 300 μm gemahlen werden kann. Die erhaltene pulverförmige katalytisch aktive Masse kann in an sich üblicher Weise ggf. unter Zusatz von pulverförmigen Trägerstoffen, wie beispielsweise Siliziumdioxid zu geformten Katalysatorteilchen, z. B. Zylinderchen eines Durchmessers von 3 bis 7 und einer Länge von 3 bis 7 mm oder zum Beschichten von Trägerkugeln eines Durchmessers von 2 bis 7 mm oder auch von Trägerringen eingesetzt werden.

Die nach dem neuen Verfahren hergestellten katalytisch aktiven Massen bzw. die daraus hergestellten Katalysatoren können mit Vorteil für die Gasphasenoxidation von Olefinen, wie insbesondere Propylen sowie ferner Isobutylen unter den hierfür üblichen Bedingungen zu den entsprechenden α, β-monoolefinisch ungesättigten Aldehyden, wie besonders Acrolein, eingesetzt werden. Man arbeitet dabei im allgemeinen bei Temperaturen von 250 bis 450, insbesondere von 300 bis 380°C und Drucken von 1,0 bis 3, insbesondere von 1,1 bis 1,8 bar, wobei die Zusammensetzung des Gasgemisches im Bereich von 2 bis 15, insbesondere von 5 bis 10 Vol.% Propylen liegt.

Es ist ein Vorteil bei dem neuen Verfahren zur Herstellung der katalytisch aktiven Massen, daß eine besondere homogene Verteilung der einzelnen Katalysatorkomponenten erreicht wird.

Beim Einsatz der neuen katalytisch aktiven Massen für die Gasphasenoxidation von Olefinen, wie besonders Propylen, wird eine verbesserte Selektivität nach Acrolein plus Acrylsäure und eine deutlich verbesserte Aktivität erzielt.

Die in den folgenden Beispielen angegebenen Teile und Prozente beziehen sich, wenn nicht anders angegeben, auf das Gewicht.

Beispiel 1

a) Eine katalytisch aktive Masse der allgemeinen Formel :

$$Mo_{12} Bi_{1 \cdot 2} W_{2 \cdot 4} Co_{4 \cdot 8} Fe_{0 \cdot 8} Si_{1 \cdot 6} K_{0 \cdot 05} O_x$$

wird wie folgt hergestellt :

476,70 g Ammoniumheptamolybdat werden mit destilliertem Wasser, das mit 11,25 ml 1n KOH versetzt ist, in der Wärme gelöst. Nach dem Abkühlen der Lösung werden unter Rühren 79,75 g $NH_4NO_3$-haltiges Eisenmolybdat, 349,23 g Kobaltnitrat, 209,23 g Wismutwolframat ($Bi_{1,2} W_{2,4} O_x$) und 80,11 g einer 30 %igen Kieselsol-Lösung nacheinander zugegeben. Die Suspension wird weiter gerührt, bis sie sich nach 10 bis 15 Minuten zu einem körnigen Brei verfestigt. Dieser wird 16 Stunden bei 110°C in einer Porzellanschale getrocknet und anschließend auf eine Korngröße von 0,71 bis 1,60 mm gesplittet. Der Splitt wird 6 Stunden bei 450°C im Drehrohr kalziniert.

b) 40 Teile des nach (a) hergestellten Splits werden in ein Rohr eines freien Durchmessers von 0,8 cm und einer Länge von 1,0 m eingefüllt und darüber bei 360°C ein Gemisch aus Propylen/Luft/Stickstoff/Wasser im Verhältnis von 1 : 9 : 10 : 2 geleitet. Bei einer Strömungsgeschwindigkeit von 76 Volumenteilen/Stunde wird eine Ausbeute an Acrolein plus Acrylsäure von 90,6 Mol-%, bezogen auf das eingesetzte Propylen, erzielt.

Vergleichsbeispiel 1

a) Ein Katalysator der unter Beispiel 1a angegebenen Zusammensetzung wird wie folgt hergestellt :
Eine wäßrige Lösung mit 211,9 g Ammoniumheptamolybdat und 5 ml 0,1 N KOH wird vorgelegt und auf 40°C erwärmt. Dazu wird koninuierlich eine Lösung aus 139,69 g Kobaltnitrat und 32,32 g Eisennitrat zugegeben. Zum Abschluß gibt man noch 19,79 g einer 48,6%igen Kieselsol-Lösung zu.

Die Suspension wird anschließend versprüht und 180 g mit einer Korngröße < 0,160 mm mit 47,48 g Wismutwolframat gemischt und mit Wasser geknetet.

Die Knetmasse wird 16 Stunden getrocknet und auf eine Korngröße von 0,71 bis 1,60 mm gesplittet.

Der Splitt wird 6 Stunden bei 450°C im Drehrohr kalziniert.

Der Katalysator wird dann, wie bei Beispiel 1 b) angegeben, bei 360°C getestet, wobei eine Ausbeute an Acrolein (plus Acrylsäure) von maximal 85 Mol-% erzielt wird.

## Beispiel 2

a) Man stellt einen Katalysator der allgemeinen Formel

$$MO_{12} Bi_{0,98}Fe_{1,98} Ni_{8,34} Zn_{1,95} K_{0,05} Na_{0,15} Si_{10} O_x,$$

wie folgt her:

66,76 g Ammoniumheptamolybdat werden mit 67 g Wasser in der Wärme gelöst. Nach dem Abkühlen gibt man unter Rühren 76,82 g Nickelnitrat und eine salpetersaure Lösung aus 21,24 g Zinknitrat und 19,85 g Wismutnitrat zu. Zum Schluß fügt man noch 39,91 g NH$_4$NO$_3$-haltiges Eisenmolybdat, 2,08 ml KOH 1n sowie 51,52 g einer 48,6 %igen Kieselsol-Lösung zu.

Nach kurzem Rühren und leichtem Erwärmen verfestigt sich die Suspension zu einem körnigen Brei. Dieser wird 16 Stunden bei 110°C in einer Porzellanschale getrocknet, auf eine Korngröße von 0,71 bis 1,60 mm gesplittet und 2 Stunden bei 580°C im Drehrohr unter Luft kalziniert.

b) Der Katalysator wird wie bei Beispiel 1 b) angegeben getestet und bei einer Temperatur von 360°C eine Ausbeute an Acrolein (plus Acrylsäure) von 87 Mol-% erzielt.

## Beispiel 3

a) Ein Katalysator der allgemeinen Formel

$$Mo_{12} Bi_{0,98} Ni_{8,29} Fe_{1,95} Si_{10} K_{0,05} Na_{0,15} O_x,$$

wird, wie in Beispiel 2 angegeben, hergestellt, jedoch anstelle des Zinknitrats eine erhöhte Menge Nickelnitrat, nämlich insgesamt 100,45 g Nickelnitrat eingesetzt.

b) Der Katalysator wird, wie bei Beispiel 1 b) angegeben, getestet und dabei eine Ausbeute von 81 Mol-% Acrolein (plus Acrylsäure) bei 360°C und einer Gasgeschwindigkeit von 80 Volumenteilen/Stunde erzielt.

## Patentansprüche

1. Verfahren zur Herstellung einer für die Gasphasenoxidation von Propylen zu Acrolein und Acrylsäure katalytisch aktiven Masse der allgemeinen Formel

$$Mo_{12}Bi_{0,1-10}Fe_{0,8-12}[Co,Ni]_{0,1-10}P_{0-2}A_{0-10}B_{0,001-1}O_x$$

in der

A für die Elemente Arsen, Antimon, Zinn, Thallium, Wolfram, die Erdalkalimetalle, Zink und/oder Chrom

B für mindestens eines der Metalle Natrium, Kalium, Rubidium, Cäsium und/oder Indium und

X für die durch die Atomwertigkeiten der Einzelelemente bestimmte Zahl

stehen, durch Vermischen von Salzen der Bestandteile in wäßrigen Medium, Abdampfen des Wassers und Kalzinieren bei Temperaturen von 400 bis 650°C, dadurch gekennzeichnet, daß man ein getrocknetes pulverförmiges Eisenmolybdat-Gel (Fe(MoO$_4$)$_3$) zu einer wäßrigen Lösung oder Suspension von hierfür üblichen wasserlöslichen Salzen der anderen Bestandteile, einschließlich eines eventuellen Überschusses an Eisen- oder Molybdänsalz über die durch das Eisenmolybdat eingetragene Menge gibt und das Gemisch unter Rühren und eventuellem Erwärmen zum gelartigen Erstarren bringt, das Gel zerkleinert und bei erhöhter temperatur trocknet und dann kalziniert.

2. Verwendung der nach Anspruch 1 hergestellten katalytisch aktiven Masse nach deren Verformung als Katalysator für die Gasphasenoxidation von Propylen zu Acrolein und Acrylsäure unter üblichen Bedingungen von Druck, Temperatur und Gaszusammensetzung.

## Claims

1. A process for preparing, for the gas phase oxidation of propylene to acrolein and acrylic acid, a catalytically active composition of the general formula

$$Mo_{12}Bi_{0.1-10}Fe_{0.8-12}[Co,Ni]_{0.1-10}P_{0-2}A_{0-10}B_{0.001-1}O_x$$

where

A is arsenic, antimony, tin, thallium, tungsten, an alkaline earth metal, zinc and/or chromium,

B is at least one of the metals sodium, potassium, rubidium, cesium and/or indium and

X is the number determined by the atomic valences of the individual elements,

by mixing salts of the constituents in an aqueous medium, evaporating the water and calcining at from 400 to 650°C, wherein a dried pulverulent iron molybdate gel (Fe(MoO$_4$)$_3$) is added to an aqueous solution or suspension of watersoluble salts of the other constituents customary for this purpose, including any excess of iron or molybdenum salt beyond the amount introduced with the iron molybdate, the mixture is rigidified into a gel by stirring and possibly heating, and the gel is comminuted and dried at an elevated temperature and then calcined.

2. Use of the catalytically active composition as prepared in claim 1, after it has been molded, as a catalyst for the gas phase oxidation of propylene to acrolein and acrylic acid under customary pressure, temperature and gas composition conditions.

**Revendications**

1. Procédé de préparation d'une masse catalytiquement active pour l'oxydation en phase gazeuse du propylène en acroléine et acide acrylique, ladite masse répondant à la formule générale

$$Mo_{12}Bi_{0,1-10}Fe_{0,8-12}[Co,Ni]_{0,1-10}P_{0-2}A_{0-10}B_{0,001-1}O_x$$

dans laquelle

A est mis pour les éléments arsenic, antimoine, étain, thallium, tungstène, les métaux alcalino-terreux, le zinc et/ou le chrome,

B est mis pour au moins l'un des métaux sodium, potassium, rubidium, césium et/ou indium et

x est mis pour le nombre déterminé par les valences des éléments individuels,

par mélange, de sels des constituants en milieu aqueux, évaporation de l'eau et calcination à des températures de 400 à 650°C, caractérisé en ce que l'on ajoute un gel de molybdate de fer ($Fe(MoO_4)_3$) séché pulvérulent à une solution ou une suspension aqueuse de sels solubles dans l'eau usuels à cet effet des autres constituants, y compris un excès éventuel de sel de fer ou de molybdène par rapport à la quantité apportée par le molybdate de fer et on amène le mélange à se solidifier sous forme d'un gel par agitation et éventuellement par chauffage, on broie le gel et on le sèche à température élevée, puis on le calcine.

2. Utilisation de la masse catalytiquement active préparée suivant la revendication 1 après qu'elle a été mise sous forme de catalyseur, pour l'oxydation en phase gazeuse du propylène en acroléine et acide acrylique dans les conditions usuelles de pression, température et composition du gaz.